# EUROPEAN PATENT SPECIFICATION

(11) **EP 1 790 360 B1**
(45) Date of publication and mention of the grant of the patent: **08.01.2014**
(21) Application number: 05025903.5
(22) Date of filing: 28.11.2005
(51) Int. Cl.: B03C 3/38, F24F 3/16, A61L 9/22, H01T 23/00

(54) **Sterilizing method**
Verfahren zur Sterilisation
Méthode de stérilisation,

(43) Date of publication of application: 30.05.2007
(60) Divisional application: 08020908.3
(73) Proprietor: Samsung Electronics Co., Ltd., Suwon-si, Gyeonggi-do, 443-742 (KR)
(72) Inventor: Park, Eun Rae, Yeongtong-Dong, Suwon-Si, Gyeonggi-Do (KR); Kwon, Jun Hyoun, Gangnam-gu, Seoul (KR)
(74) Representative: Grünecker, Kinkeldey, Stockmair & Schwanhäusser

(56) References cited:
- EP-A- 1 625 890
- EP-A1- 1 791 232
- WO-A-2005/077523
- JP-A- 2004 138 268
- US-A- 3 942 072
- US-B1- 6 350 417
- US-B1- 6 850 403
- PATENT ABSTRACTS OF JAPAN vol. 2003, no. 12, 5 December 2003 (2003-12-05) & JP 2005 116229 A (SHARP CORP), 28 April 2005 (2005-04-28)

## Description

### BACKGROUND OF THE INVENTION

### 1. Field of the Invention

The present invention relates to a sterilizing method using an ion generating apparatus.

### 2. Description of the Related Art

Generally, an air cleaning apparatus includes a filter mounted in a housing for filtering diverse impurities; a blowing fan for introducing air from a room space into the housing, thereby forcing the introduced air to pass through the filter, and for discharging the filtered air out of the housing; and an anion generator for generating anions.

JP 2004-138268 refers to emitting electrons in a straight line from a needle electrode generated by corona discharge, and distantly delivering a generated ionic wind.

EP 1 625 890 A2 discloses an ion generating element and an ion generating apparatus that, by releasing positive and negative ions into a space, can decompose bacteria, mold spores, toxic substances, and the like floating in the air.

EP 1 791 232 A1 which was published after the application date of the present invention discloses an ion generating apparatus and an air cleaning apparatus using the same, and, more particularly, an ion generating apparatus for generating anions and cations, and an air cleaning apparatus using the same.

In this air cleaning apparatus, the room air is cleaned while passing through the filter when the blowing fan is driven, and is then discharged into the room space, together with anions generated from the anion generator. However, such a conventional air cleaning apparatus, which includes a filter and an anion generator, is limited in its ability to kill bacteria floating in the air, using only the filter and anions generated from the anion generator. In order to solve this problem, a new ion generator has been proposed which performs sterilization using both anions and cations. Such an ion generator, which can generate both anions and cations, is described in Japanese Patent Laid-open Publication No. 2003-123940.

In this conventional ion generator, AC voltage is applied to two electrodes to alternately generate anions and cations. The generated anions and cations are supplied into a room space. In this case, the cations are hydrogen ions (H⁺), and the anions are super-oxide anions (O₂⁻). When the hydrogen ions and super-oxide anions are supplied into the room space, they produce hydroxyl radicals (OH) or hydrogen peroxide (H₂O₂). These compounds are adsorbed to bacteria present in the room air, to thereby oxidize the bacteria and kill the bacteria.

However, this conventional ion generator has a problem in that the hydrogen ions, which are harmful to the human body, are discharged into the room space intact, so that the user's health may be negatively impacted upon inhaling the hydrogen ions.

Furthermore, since each electrode alternately generates anions and cations, considerable amounts of anions and cations are coupled before they can be used for sterilization, so that their effectiveness is lost.

In addition, since each electrode alternately generates anions and cations, it is impossible to generate sufficient amounts of anions and cations required for sterilization within a short period of time.

### SUMMARY OF THE INVENTION

The invention is defined according to the independent claim 1.

Additional aspects and/or advantages of the invention will be set forth in the detailed description which follows.

### BRIEF DESCRIPTION OF THE DRAWINGS

These and/or other aspects and advantages of the invention will become more apparent from the following description of the embodiments, taken in conjunction with the accompanying drawings in which:
FIG. 1 is a perspective view illustrating a sterilizing apparatus;
FIG. 2 is a schematic view showing ions generated from the sterilizing apparatus of FIG. 1;
FIGS. 3a to 3e are schematic views showing a sterilizing method carried out by the sterilizing apparatus of FIG. 1;
FIG. 4 is a schematic view illustrating a testing apparatus for testing the performance of the sterilizing apparatus shown in FIG. 1;
FIG. 5 shows graphs depicting the test results obtained by the testing apparatus of FIG. 4;
FIG. 6 is a perspective view illustrating a sterilizing apparatus;
FIG. 7 is a schematic view showing ions generated from the sterilizing apparatus of FIG. 6; and
FIG. 8 is a schematic view illustrating an air cleaning apparatus in which the sterilizing apparatus of FIG. 1 is installed.

### DETAILED DESCRIPTION OF THE PREFERRED EMBODIMENTS

Reference will now be made in detail to the embodiments of the sterilizing apparatus, examples of which are illustrated in the accompanying drawings, wherein like reference numerals refer to the like elements throughout. The embodiments are described below to explain the method of the present invention by referring to the figures. However, the present invention should not be construed as being limited thereto.

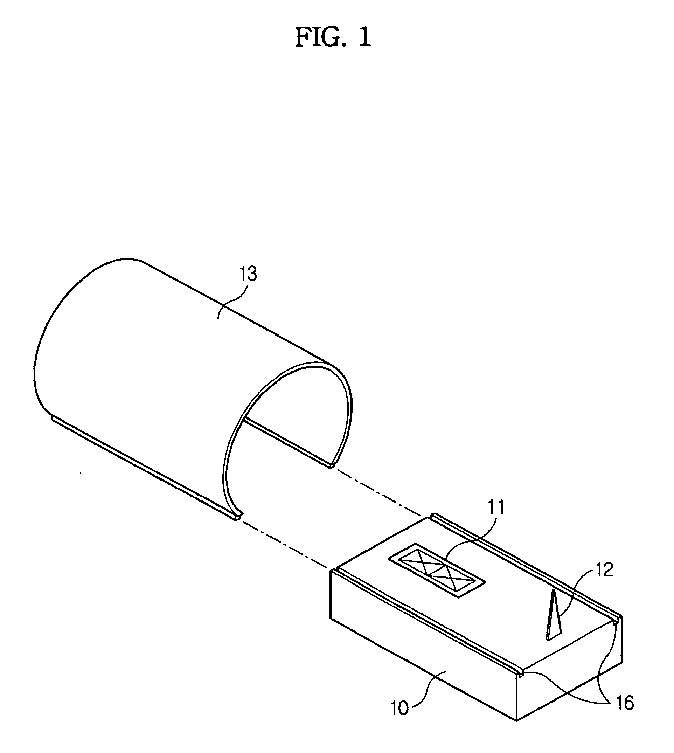

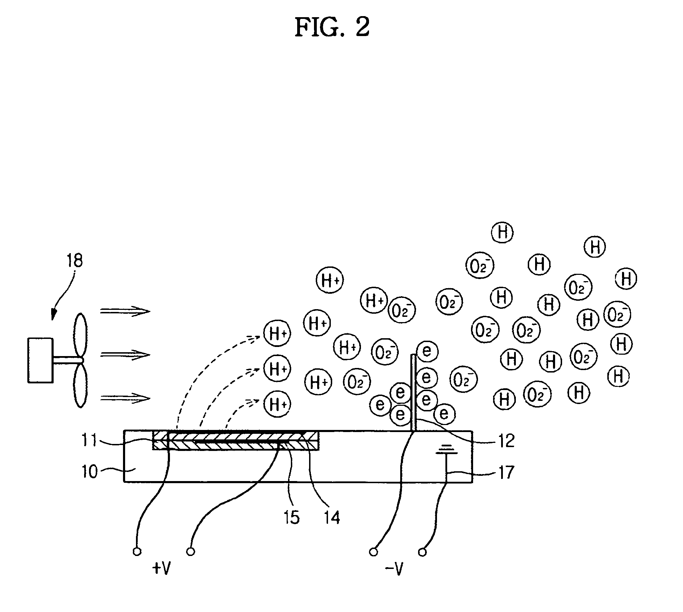

FIGS. 1 and 2 illustrate a sterilizing apparatus. As shown in FIGS. 1 and 2, the sterilizing apparatus includes a base 10, a ceramic plate 11 mounted on an upper surface of the base 10, a needle-shaped electrode 12 mounted on the base 10 while being spaced apart from the ceramic plate 11 by a predetermined distance, and a cover 13 which defines the diffusion range of ions generated from the ceramic plate 11 and needle-shaped electrode 12 within a predetermined space.

A recess is provided at the upper surface of the base 10 for mounting the ceramic plate 11. The ceramic plate 11 is fitted in the base 10. The ceramic plate 11 is adapted for generating cations. As shown in FIG. 2, a discharge electrode 14 is provided at an upper surface of the ceramic plate 11 inside the ceramic plate 11. An induction electrode 15 is also provided at a middle portion within the ceramic plate 11 when viewed in a thickness direction of the ceramic plate 11. The remaining portion of the ceramic plate 11 is made of ceramic to form a protective layer.

A high positive voltage is applied between the discharge electrode 14 and the induction electrode 15. The high positive voltage is preferably 3.9kV to 4.3kV, even though it may have other voltage ranges. When such a high positive voltage is applied between the discharge electrode 14 and the induction electrode 15, plasma discharge occurs at the ceramic plate 11, so that moisture (H₂O) present in the air around the ceramic plate 11 is ionized, thereby generating hydrogen ions (H⁺).

Meanwhile, a high negative voltage is applied between the needle-shaped electrode 12 and a ground electrode 17. The high negative voltage is preferably -3.2kV to -3.6kV, even through it may have other voltage ranges. When such a high negative voltage is applied between the needle-shaped electrode 12 and the ground electrode 17, plasma discharge occurs at the needle-shaped electrode 12, so that cations are accumulated around the needle-shaped electrode 12, and a large amount of electrons are discharged from the needle-shaped electrode 12 into the air. The electrons discharged into the air are very unstable and therefore are captured by oxygen molecules (O₂) in the air, thereby forming super-oxide anions (O₂⁻). Thus, when a high negative voltage is applied to the needle-shaped electrode 12, electrons and super-oxide anions are generated.

Electrons discharged from the needle-shaped electrode 12 are coupled with hydrogen ions reaching the needle-shaped electrode 12 after being generated from the ceramic plate 11, so that hydrogen atoms (or active hydrogen) are produced. In order to promote coupling of the hydrogen ions, generated from the ceramic plate 11, with the electrons generated from the needle-shaped electrode 12, a blower 18 may be arranged at one side of the sterilizing apparatus to forcibly feed the hydrogen ions toward the needle-shaped electrode 12.

As described above, the needle-shaped electrode 12 is spaced apart from the ceramic plate 11 by a predetermined distance. It is desirable to appropriately determine the spacing between the ceramic plate 11 and the needle-spaced electrode 12, based on the size of the ceramic plate 11 and the height of the needle-shaped electrode 12. This is because the amount of hydrogen atoms formed from the hydrogen ions generated from the ceramic plate 11 varies depending on the spacing between the ceramic plate 11 and the needle-shaped electrode 12.

Since the hydrogen ions generated from the ceramic plate 11 are coupled with the electrons discharged from the needle-shaped electrode 12, and thus, form hydrogen atoms, hydrogen atoms and super-oxide anions are finally discharged from the sterilizing apparatus.

The cover 13 has a tunnel-shaped structure opened at the bottom thereof to have a pair of laterally-spaced longitudinal lower ends. The cover 13 is separably coupled with the base 10 in such a manner that the lower ends of the cover 13 are slidably engaged with cover rails 16 formed at lateral ends of the base 10 on the upper surface of the base 10, respectively. When the sterilizing apparatus generates hydrogen ions while blowing air into the cover 13 at one side of the cover 13 under the conditions in which the cover 13 is coupled with the base 10, the hydrogen ions thus generated are moved toward the needle-shaped electrode 12 within the cover 13 and the hydrogen ions are then coupled with electrons discharged from the needle-shaped electrode 12, thereby producing hydrogen atoms which are, in turn, discharged from the other side of the cover 13. Together with the hydrogen atoms, super-oxide anions generated from the needle-shaped electrode 12 are also discharged from the other side of the cover 13 by the blowing air.

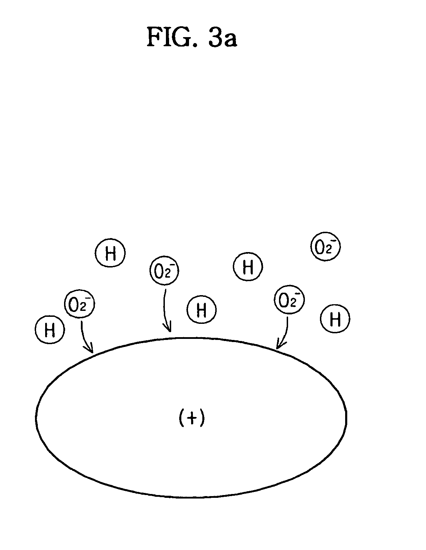

Hereinafter, a sterilizing method carried out by the sterilizing apparatus of FIG. 1 will be described with reference to FIGS. 3a to 3e.

In accordance with the sterilizing method, a high positive voltage is first applied between the discharge electrode 14 and induction electrode 15 in the ceramic plate 11, thereby generating hydrogen ions. Simultaneously, a high negative voltage is applied to the needle-shaped electrode 12, thereby generating electrons and super-oxide anions. Also, air is blown into the cover 13 at one side of the cover 13 to feed the hydrogen ions to the needle-shaped electrode 12 or a region around the needle-shaped electrode 12.

When the hydrogen ions approach the needle-shaped electrode 12, they are coupled with the electrons present around the needle-shaped electrode 12, thereby producing hydrogen atoms. The hydrogen atoms are then discharged from the cover 13, together with the super-oxide anions generated from the needle-shaped electrode 12.

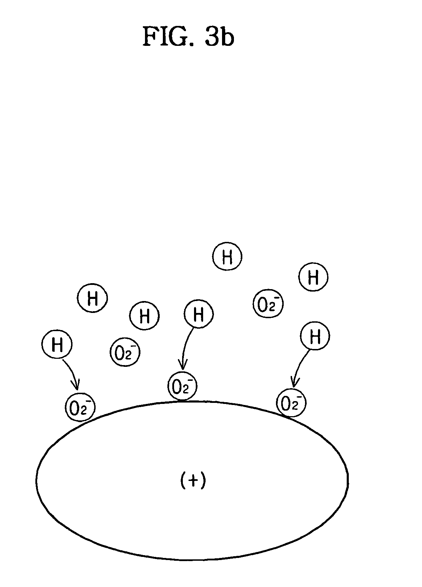

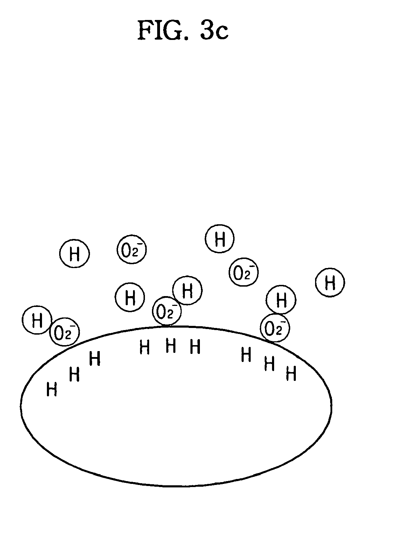

The super-oxide anions, which are discharged from the sterilizing apparatus into the air, exhibit a polarity opposite to static electricity (+) of bacteria floating in the air, so that the super-oxide anions are adsorbed to the surface of the bacteria, as shown in FIG. 3a. Once the super-oxide anions are adsorbed to the surface of the bacteria, the hydrogen atoms, which are discharged from the sterilizing apparatus into the air, react with the super-oxide anions, as shown in FIGS. 3b and 3c.

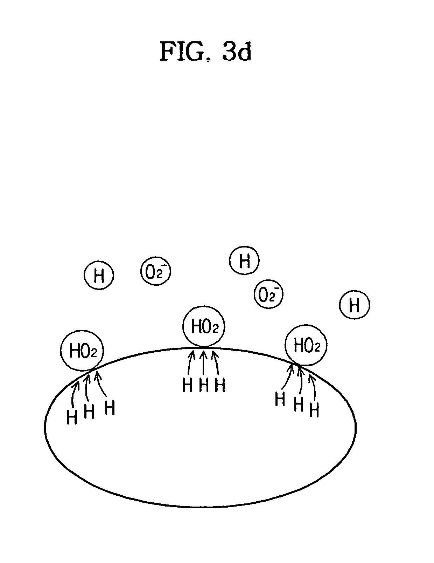

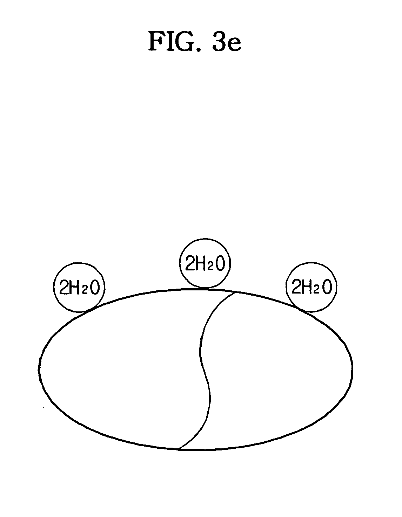

When such a reaction is carried out between the super-oxide anions and the hydrogen atoms, the following reactions are sequentially carried out, so that states of FIGS. 3d and 3e are sequentially obtained.

H + O₂⁻ → HO₂ (hydroperoxy radical) + e + Static Electricity of Bacteria

HO₂ + 3H (hydrogen atoms of protein forming a cell membrane of the bacteria) → 2H₂O

That is, the super-oxide anions and hydrogen atoms, which react with each other, form hydroperoxy radicals. Also, the electrons of the super-oxide anions offset the static electricity of the bacteria. Each hydroperoxy radical then captures three hydrogen atoms from the protein forming the cell membrane of the bacteria, thereby producing two water molecules. As a result, the protein molecules of the cell membrane, which donate the hydrogen atoms, are destroyed, thereby causing the cell membrane of the bacteria to be destroyed. Thus, sterilization is achieved.

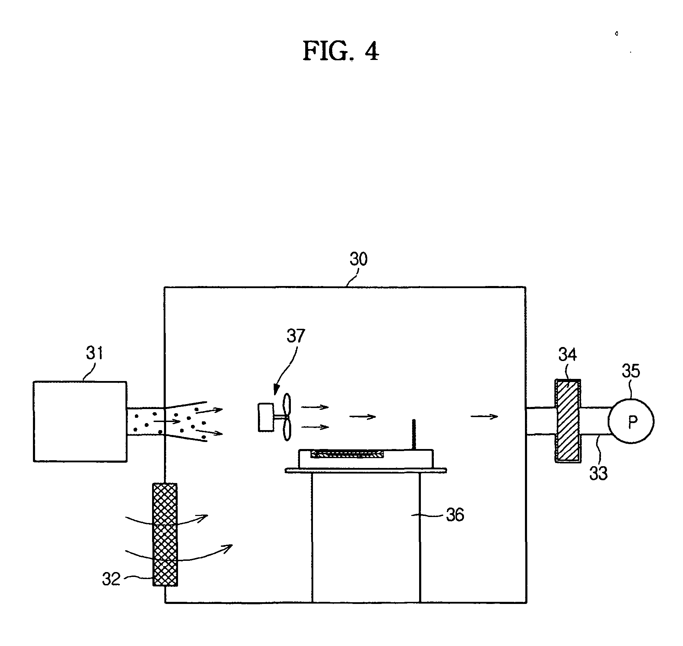

Referring to FIG. 4, an apparatus for testing the performance of the sterilizing apparatus shown in FIG. 1 is illustrated. The testing apparatus includes a chamber 30, a virus supplier 31 arranged at one side wall of the chamber 30, an ambient air sucking filter 32 arranged at one side wall of the chamber 30 beneath the virus supplier 31, and a discharge duct 33 connected to the other side wall of the chamber 30. A detection filter 34 is arranged in the discharge duct 33. A suction pump 35 is mounted to an outlet end of the discharge duct 33. A table 36, on which the sterilizing apparatus is to be laid for testing thereof, is arranged in the chamber 30. A blowing fan 37 is also arranged in the chamber 30 to blow air to the sterilizing apparatus.

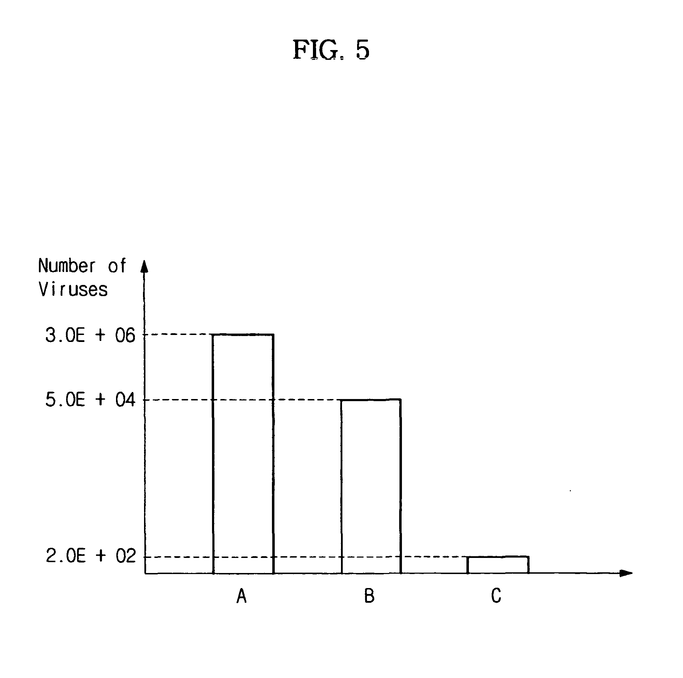

For testing, the sterilizing apparatus is first laid on the table 36 in the testing apparatus. Thereafter, viruses (Influenza viruses, New Caledonia) are supplied from the virus supplier 31 into the chamber 30 for 10 minutes. After completion of the virus supply from the virus supplier 31, the sterilizing apparatus and blowing fan 37 are driven for 30 minutes. Subsequently, the suction pump 35 is driven. In this state, the number of viruses detected by the detection filter 34 is checked. FIG. 5 shows graphs respectively depicting the number of supplied viruses and the number of viruses detected by the detection filter 34.

In FIG. 5, the Y-axis represents the number of viruses (log scale), the graph A represents the number of viruses supplied from the virus supplier 31, the graph B represents the number of viruses detected after 30 minutes without driving the sterilizing apparatus, and the graph C represents the number of viruses detected after 30 minutes of driving the sterilizing apparatus. Referring to FIG 5, it can be seen that the number of viruses was reduced when the sterilizing apparatus was not driven, however, the number of naturally reduced viruses was small. On the other hand, 99.6% of the supplied viruses are removed within 30 minutes when the sterilizing apparatus is driven.

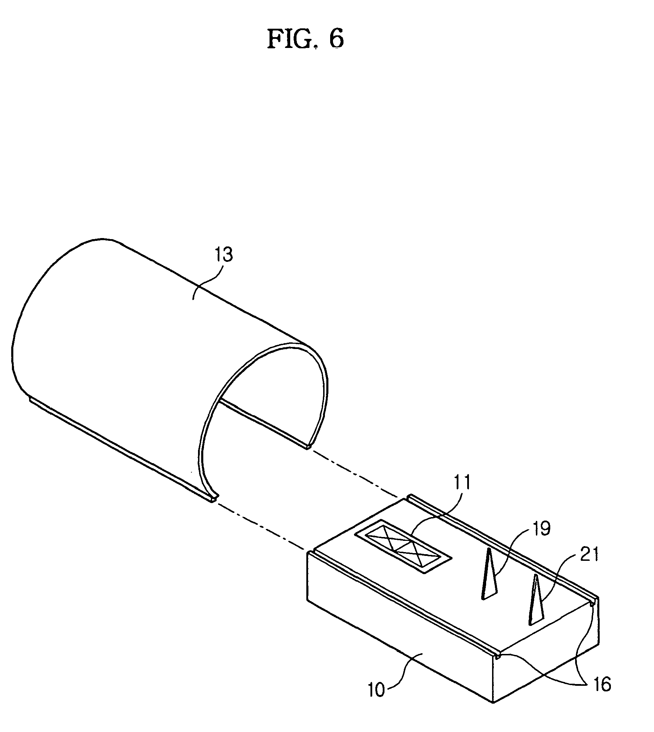

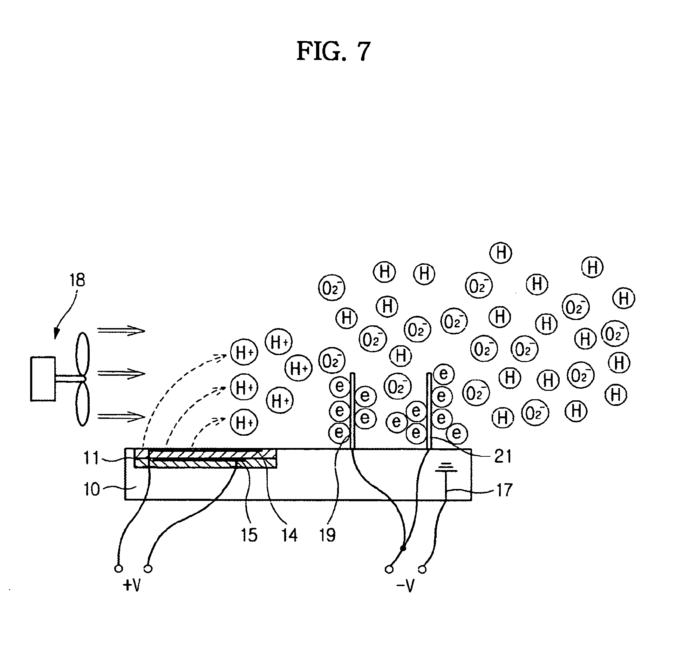

Referring to FIGS. 6 and 7, a sterilizing apparatus according to an illustrative example not being part of the present invention. In FIGS. 6 and 7, constituent elements respectively corresponding to those in FIGS. 1 and 2 are denoted by the same reference numerals. As shown in FIGS. 6 and 7, the sterilizing apparatus includes a base 10, a ceramic plate 11 mounted on an upper surface of the base 10, a first needle-shaped electrode 19 mounted on the base 10 while being spaced apart from the ceramic plate 11 by a desired distance to generate electrons, a second needle-shaped electrode 21 mounted on the base 10 while being spaced apart from the first needle-shaped electrode 19 by a desired distance to generate super-oxide anions, and a cover 13 to define the diffusion range of ions generated from the ceramic plate 11 and needle-shaped electrodes 19 and 21 within a predetermined space. The ceramic plate 11 and cover 13 of FIG. 6 are the same as those of FIGS. 1 and 2, so that no description thereof will be given.

A high negative voltage of, for example, -3.2kV to -3.6kV, is applied between each of the first and second needle-shaped electrodes 19 and 21 and a ground electrode 17. When such a high negative voltage is applied between the first needle-shaped electrode 19 and the ground electrode 17, plasma discharge occurs at the first needle-shaped electrode 19, so that cations accumulate around the first needle-shaped electrode 19, and a large amount of electrons are discharged from the first needle-shaped electrode 19 into the air.

Since the first needle-shaped electrode 19 is arranged in the vicinity of the ceramic plate 11, the electrons discharged from the first needle-shaped electrode 19 are coupled with hydrogen ions reaching the first needle-shaped electrode 19 after being generated from the ceramic plate 11, so that hydrogen atoms (or active hydrogen) are produced. In order to promote the coupling of the hydrogen ions, generated from the ceramic plate 11, with the electrons generated from the first needle-shaped electrode 19, a blower 18 may be arranged at one side of the sterilizing apparatus to forcibly feed the hydrogen ions toward the first needle-shaped electrode 19.

Meanwhile, when the high negative voltage is applied between the second needle-shaped electrode 21 and the ground electrode 17, plasma discharge also occurs at the second needle-shaped electrode 21, so that a large amount of electrons are generated from the second needle-shaped electrode 21. Since the electrons discharged from the second needle-shaped electrode 21 into the air are very unstable, they are captured by oxygen molecules (O₂) in the air, thereby forming super-oxide anions (O₂⁻).

Thus, the hydrogen ions discharged from the ceramic plate 11 are coupled with the electrons generated from the first needle-shaped electrode 19, thereby producing hydrogen atoms. The hydrogen atoms, in turn, react with the super-oxide anions generated from the second needle-shaped electrode 21, thereby producing hydroperoxy radicals having a sterilizing function. In accordance with the second example, hydrogen atoms are produced, using electrons generated from the first needle-shaped electrode 19, and super-oxide anions are generated from the second needle-shaped electrode 21, as compared to the first example.

As described above, the first and second needle-shaped electrodes 19 and 21 are spaced apart from the ceramic plate 11 by predetermined distances, respectively. It is desirable to appropriately determine the spacing between the ceramic plate 11 and each needle-spaced electrode, based on the size of the ceramic plate 11 and the height of the needle-shaped electrode, to achieve maximal hydrogen atom producing performance.

Although the illustrative example has been described in terms of a method in which a high negative voltage is applied to both the first and second needle-shaped electrodes 19 and 21, the high negative voltage may be applied to the first needle-shaped electrode 19 alone. In this case, hydrogen atoms, which are generated from the first needle-shaped electrode 19, are coupled with super-oxide anions, which are a kind of active oxygen naturally generated in the air, thereby removing the super-oxide anions.

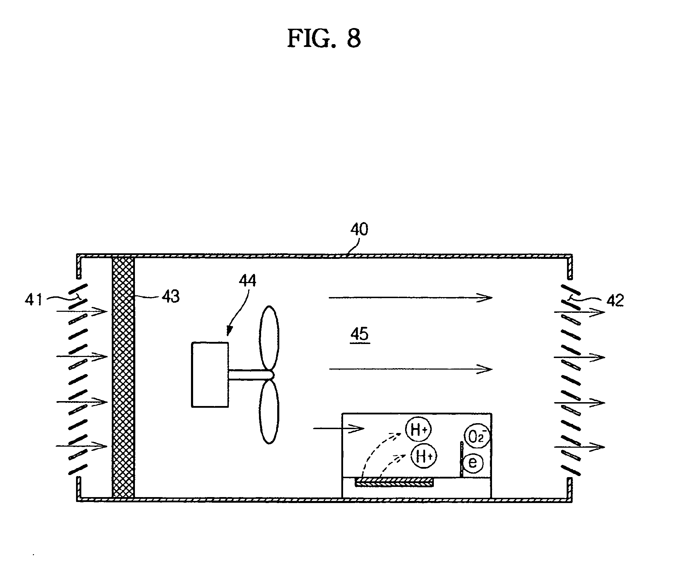

Referring to FIG. 8, an air cleaning apparatus is illustrated which uses the sterilizing apparatus of FIG. 1. The air cleaning apparatus includes a body 40, an inlet 41 arranged at one side wall of the body 40, an outlet 42 arranged at the other side wall of the body 40, a filter 43 mounted in the body 40, a blower 44 to forcibly circulate room air such that the room air is discharged out of the body 40 after passing through the filter 43, and the sterilizing apparatus of FIG. 1 arranged in an air discharge path 45 defined in the body 40.

When the blower 44 is driven to clean the room air in the air cleaning apparatus, the room air is sucked into the interior of the body 40 through the inlet 41, and then passes through the filter 43, so that impurities are removed from the room air by the filter 43. Thereafter, the room air is fed along the air discharge path 45 to be discharged out of the body 40 through the outlet 42. In this case, the sterilizing apparatus may be driven, simultaneously with the driving of the blower 44. When the sterilizing apparatus is driven, hydrogen atoms and super-oxide anions are discharged into the air discharge path 45. Accordingly, bacteria present in the room air passing through the air discharge path 45 are removed in accordance with the above-described sterilizing process using the generated hydrogen atoms and super-oxide anions.

Although this embodiment illustrates an air cleaning apparatus, to which the sterilizing apparatus is applied, the sterilizing apparatus of FIG. 1 and the sterilizing method using the same may also be applied to appliances requiring sterilization, other than air cleaners, for example, air conditioners, ventilators, and refrigerators.

Also, although hydrogen atoms and super-oxide anions are distributed into the air so that they are coupled in the air to produce hydroperoxy radicals for sterilization, in the illustrated examples, the hydroperoxy radicals themselves may be distributed into the air. In the latter case, the hydrogen atoms and super-oxide anions are coupled in the sterilizing apparatus to produce hydroperoxy radicals which are, in turn, distributed into the air.

In both examples, the cover 13 is used to promote the reaction between hydrogen ions and electrons and the reaction between hydrogen atoms and super-oxide anions. However, the present invention may be implemented without using the cover 13.

As apparent from the above description, hydrogen atoms are used for sterilization, in place of hydrogen ions harmful to the human body. Accordingly, it is possible to prevent the user from being exposed to the harmful hydrogen ions during the sterilization.

The sterilizing apparatus includes a cation generator and an anion generator, which are separated from each other. Accordingly, it is possible to prevent a reduction in the number of ions available for sterilization due to ion loss caused by coupling of cations and anions prior to the use thereof for sterilization, as in conventional cases in which cations and anions are alternately generated from the same ion generator.

In addition, since the cation generator and anion generator are separated from each other, it is possible to produce a sufficient amount of ions required for sterilization, and thus, to achieve an enhancement in sterilizing performance.

## Claims

1. A sterilizing method using a sterilizing apparatus comprising: a first electrode, a second electrode, and a blower (18);
the method comprising:
generating hydrogen ions in the air by the first electrode;
generating electrons and super-oxide anions by the second electrode,
feeding the hydrogen ions generated from the first electrode toward the electrons discharged from the second electrode by means of the blower (18);
wherein the second electrode is arranged such that the hydrogen ions generated from the first electrode react with the electrons generated from the second electrode, thereby producing hydrogen atoms, which, in turn, react with the super-oxide anions generated from the second electrode, thereby killing bacteria present in the air;
wherein the first electrode comprises a ceramic plate (11), a discharge electrode (14) and an induction electrode (15), between which a high positive voltage is to be applied;
wherein said discharge electrode (14) is provided at an upper surface of said ceramic plate (11) inside said ceramic plate (11) and said induction electrode (15) is provided at a middle portion within said ceramic plate (11) such that plasma discharge occurs at the ceramic plate (11), so that moisture present in the air around the ceramic plate is ionized, thereby generating hydrogen ions; and
wherein the second electrode comprises a needle-shaped electrode (12) and a ground electrode (17), between which a high negative voltage is to be applied.

## Patentansprüche

1. Verfahren zur Sterilisierung unter Verwendung einer Sterilisierungsvorrichtung mit einer ersten Elektrode, einer zweiten Elektrode und einem Blaslüfter (18); das Verfahren umfassend:
Erzeugen von Wasserstoffionen in der Luft durch die erste Elektrode;
Erzeugen von Elektronen und Superoxid-Anionen durch die zweite Elektrode,
Zuführen der von der ersten Elektrode erzeugten Wasserstoffionen zu den von der zweiten Elektrode freigesetzten Elektronen unter Verwendung des Blaslüfters (18);
wobei die zweite Elektrode derart angeordnet ist, dass die von der ersten Elektrode erzeugten Wasserstoffionen mit den von der zweiten Elektrode erzeugten Elektronen reagieren, wodurch Wasserstoffatome erzeugt werden, welche wiederum mit den von der zweiten Elektrode erzeugten Superoxid-Anionen reagieren, wodurch Bakterien, die in der Luft vorhanden sind, getötet werden;
wobei die erste Elektrode eine keramische Platte(11), eine Entladungselektrode (14) und
eine Induktionselektrode, zwischen denen eine hohe positive Spannung anzulegen ist, umfasst;
wobei besagte Entladungselektrode (14) an einer oberen Fläche der besagten keramischen Platte innerhalb der keramischen Platte (11) vorgesehen ist und besagte Induktionselektrode (15) in einem mittleren Abschnitt in der keramischen Platte (11) vorgesehen ist, so dass eine Plasmaentladung an der keramischen Platte (11) auftritt, so dass Feuchtigkeit, die in der die keramische Platte umgebenden Luft vorhanden ist, ionisiert wird, so dass Wasserstoffionen erzeugt werden; und
wobei die zweite Elektrode eine nadelförmige Elektrode (12) und eine Masseelektrode (17) umfasst, zwischen denen eine hohe negative Spannung anzulegen ist.

## Revendications

1. Procédé de stérilisation utilisant un appareil de stérilisation comprenant : une première électrode, une seconde électrode et une soufflante (18) ;
le procédé comprenant :
la génération d'ions d'hydrogène dans l'air par la première électrode ;
la génération d'électrons et d'anions de superoxyde par la seconde électrode,
la fourniture des ions d'hydrogène générés par la première électrode vers les électrons déchargés depuis la seconde électrode au moyen de la soufflante (18) ;
dans lequel la seconde électrode est agencée de telle sorte que les ions d'hydrogène générés par la première électrode réagissent avec les électrons générés par la seconde électrode, de façon à produire des atomes d'hydrogène, qui réagissent eux-mêmes avec les anions de superoxyde générés par la seconde électrode, tuant ainsi les bactéries présentes dans l'air ;
dans lequel la première électrode comprend une plaque en céramique (11), une électrode de décharge (14) et une électrode d'induction (15) entre lesquelles est appliquée une haute tension positive ;
dans lequel ladite électrode de décharge (14) est disposée sur la surface supérieure de ladite plaque en céramique (11) à l'intérieur de ladite plaque en céramique (11) et ladite électrode d'induction (15) est disposée dans la partie médiane de ladite plaque en céramique (11) de telle sorte qu'une décharge de plasma se produit au niveau de la plaque en céramique (11), de façon à ioniser l'humidité présente dans l'air qui entoure la plaque en céramique, générant ainsi des ions d'hydrogène ; et
dans lequel la seconde électrode comprend une électrode en forme d'aiguille (12) et une électrode de masse (17) entre lesquelles doit être appliquée une haute tension négative.
